**Europäisches Patentamt**

**European Patent Office**

**Office européen des brevets**

(19)

(11) Publication number : **0 239 790 B1**

(12) # EUROPEAN PATENT SPECIFICATION

(45) Date of publication of patent specification :
**08.04.92 Bulletin 92/15**

(51) Int. Cl.⁵ : **C01C 3/20, C08F 8/34**

(21) Application number : **87102713.2**

(22) Date of filing : **26.02.87**

(54) **Stable thiocyanogen composition and method for preparing the same.**

(30) Priority : **28.02.86 US 834983**

(43) Date of publication of application :
**07.10.87 Bulletin 87/41**

(45) Publication of the grant of the patent :
**08.04.92 Bulletin 92/15**

(84) Designated Contracting States :
**BE DE ES FR GB IT NL SE**

(56) References cited :
**GB-A- 1 108 815**
**US-A- 2 781 331**
**US-A- 3 607 536**
**US-A- 4 567 241**
**US-A- 4 613 653**

(73) Proprietor : **THE DOW CHEMICAL COMPANY**
**2030 Dow Center Abbott Road P.O. Box 1967**
**Midland Michigan 48640-1967 (US)**

(72) Inventor : **Brewster, Steven Lane**
**103 Pine**
**Lake Jackson Texas 77566 (US)**

(74) Representative : **Huber, Bernhard, Dipl.-Chem.**
**et al**
**Patentanwälte H. Weickmann, Dr. K. Fincke**
**F.A. Weickmann, B. Huber Dr. H. Liska, Dr. J.**
**Prechtel Kopernikusstrasse 9 Postfach 86 08**
**20**
**W-8000 München 86 (DE)**

EP 0 239 790 B1

## Description

It is known that thiocyanogen is an active compound useful for treating the surfaces of polymers to enhance the adhesion of paint applied thereto. Thiocyanogen is also useful in the preparation of organic thiocyanates or isothiocyanates by reacting it with organic compounds.

The reaction with the polymers or with organic compounds is advantageously conducted in solution since the thiocyanogen, under normal conditions, can not be isolated as such. Even in solution, however, the thiocyanogen is not stable and will precipitate from solution as a polymeric material which is unreactive for the purposes desired. Thus, these solutions must be used within a short period of time after preparation. Therefore, it would be highly desirable to develop a stable form of such solutions to enable them to be shipped and to be used at a time somewhat later than the time at which they are prepared.

Thiocyanogen is prepared by methods known to the art. Metal thiocyanates are oxidized, e.g. reacted with $Cl_2$, to form the thiocyanogen, $(SCN)_2$. Illustrative of this reaction is the use of potassium thiocyanates:

$$2KSCN + Cl_2 \rightarrow (SCN)_2 + 2KCl$$

The thiocyanates of metals of Groups IA, IIA, and IVA of the periodic chart are also useful. Examples are sodium, lithium, potassium, calcium and lead thiocyanates.

Oxidizing agents which can be used in place of chlorine are bromine, fluorine and organic peroxides.

A thiocyanating composition has now been discovered which has the desirable properties of the unstable thiocyanogen, but in which the tendency to polymerize is inhibited, allowing it to remain active over long periods of time.

The present invention is a composition comprising the reaction product of thiocyanogen or a thiocyanogen halide and certain $\alpha,\beta$-unsaturated ketones selected from 1,2-benzoquinone, methyl vinyl ketone and halo-derivatives of 1,2-benzoquinone or methyl vinyl ketone, e.g. o-chloranil, and solutions of the reaction product in an inert solvent.

The composition is less likely to form polythiocyanogen than thiocyanogen solutions previously known, thus remaining active for the introduction of thiocyanate functionality into organic compounds over extended periods of time.

The composition can be prepared by adding to a solution of thiocyanogen or a thiocyanogen halide in an inert solvent certain conjugated unsaturated ketones according to claim 1, i.e. the unsaturation is between the carbon atoms $\alpha$- and $\beta$- to the carbonyl group. The composition can be used in the same manner as known thiocyanogen or thiocyanogen halide solutions, but does not polymerize over an extended period of time. Thus, the product can be packaged and shipped whereas the known thiocyanogen solutions are unstable and must be used within a relatively short period of time before they polymerize and form a brick-red precipitate, thereby becoming inactive.

The composition can be used to treat the surfaces of polymer products to provide a more adherent surface for paint. Thus, polyurethane manufactured products can be treated by immersing them in a solution containing the composition. The reaction which takes place provides a surface to which paint will adhere more readily. It is especially useful to treat the surface of molded polymer products in which an internal mold release such as zinc stearate and/or silicone, has been employed, which make the surfaces of molded items especially difficult to paint. Other polymers which can be reacted with the stable thiocyanogen product of this invention to provide a more adherent surface are polypropylene, polyethylene, polystyrene, polycarbonate, polyamide, polyimide, polyester and epoxy resins.

The process of thiocyanation using the reaction products of this invention can be conducted in any manner that is known for using thiocyanogen or thiocyanogen chloride solutions to react with polymers containing unsaturation or with those which are fully saturated, respectively. Teachings concerning the treatment of surfaces with thiocyanogen are disclosed in U.S. 4,567,241 and those with thiocyanogen chloride are disclosed in U.S. 4,613,653.

The compositions of this invention can also be used to react with other simpler organic compounds to prepare thiocyanate derivatives, e.g. unsaturated aliphatics such as cyclohexane, 2,3-dimethylbut-2-ene, 1-decene, and vinyl chloride, and aromatics such as styrene.

Solvents useful as carriers for the reaction product are solvents which are inert to the reaction conditions of the polymer surface treatment and resistant to organic thiocyanate formation. Solvents which can be used are, for example, aliphatic and cycloaliphatic ethers, aliphatic and aromatic hydrocarbons, aliphatic alcohols, halogenated hydrocarbons, chlorofluorocarbons, ketones, organic acids and esters. Specific solvents include ethers such as 2-methoxyethanol, 1-methoxy-2-propanol, 1,4-dioxane, and tetrahydrofuran ethers. Aliphatic and aromatic hydrocarbons which are useful include hexane, heptane, iso-octane, cyclohexane, benzene, toluene, ethylbenzene and xylene. Useful aliphatic alcohols are methanol, ethanol and 2-propanol.

Halogenated hydrocarbons including methylene chloride, carbon tetrachloride, methylchloroform, 1,2-dichloroethane and their fluorine analogs can be used, as well as fluorocarbon compounds such as Freon-113 (1,1,2-trichlorotrifluoroethane) and Freon 11 (trichlorofluoromethane). Substituted aromatic compounds such as dichlorobenzenes and nitrobenzene, are also useful.

Ketones such as methyl ethyl ketone and methyl isobutyl ketone; organic esters such as methyl acetate, ethyl acetate, and 2-ethoxyethyl acetate; carboxylic acids such as formic acid, acetic acid, propanoic acid, and butanoic acid are also useful as carriers.

These same solvents can also be used as media in which to prepare the thiocyanating compositions. Solvents that react with the chlorine, or other oxidizing agent, are not as desirable as those which do not react with the oxidizing agent. If the solvent reacts with the oxidizing agent to any appreciable extent, more of the oxidizing agent is required, thus increasing the costs of production.

The preferred solvents for use as media in the preparation of the compositions of this invention are the halogenated hydrocarbons, while those preferred as carriers, in addition to the halogenated hydrocarbons, can be any of the aforementioned solvents, depending upon the solubility of the reaction product and the particular application to be made. The reaction for making the thiocyanogen is generally conducted at ambient temperatures (20-25°C) and the reaction to form stable reaction product of thiocyanogen and the $\alpha,\beta$-unsaturated ketone is conducted at these same temperatures. Temperatures above 50°C for either reaction are to be avoided. The molar ratio of the $\alpha,\beta$-unsaturated ketones to thiocyanogen or thiocyanogen chloride in the stable composition is at least 1:1 up to 6:1, preferably from 2:1 to 4:1 and most preferably from 2:1 to 3:1.

The following are representative examples of the preparation and use of thiocyanating compositions.

Example 1

Representative Preparation of the Stable Reaction Product of Thiocyanogen With an $\alpha,\beta$ Unsaturated Ketone

Into a flask with stirring means was placed 100 ml of reagent-grade carbon tetrachloride. Lead thiocyanate, $Pb(SCN)_2$, (7.5 g, 0.023 mole) was added while stirring the contents of the flask at room temperature (20-25°C). Bromine, $Br_2$ (3.2 g, 0.02 mole) was added with continued stirring until the color of the bromine disappeared. The $PbBr_2$ precipitate was removed from the solution by filtration. An $\alpha,\beta$-unsaturated ketone [1-2 moles per mole $(SCN)_2$] was then added to the thiocyanogen solution and this solution, containing the reaction product, was stored at ambient temperatures (optionally) under a nitrogen pad and observed periodically.

Various compounds containing similar groups and/or structures were reacted with the thiocyanogen solution in the same manner. These were stored and observed periodically. The appearance of precipitate indicated instability.

The results of all the tests are shown in the following table.

| Compound | Days Stable |
|---|---|
| o-chloranil* | >150 |
| mesityl oxide | <2 |
| catechol | <2 |
| cumene | <2 |
| styrene | <2 |
| benzoic acid | <2 |

\* 3,4,5,6-tetrachloro-1,2-benzoquinone

Equivalent for the o-chloranil are 1,2-benzoquinone and its other halogen derivatives including those with fewer chlorines and those in which the chlorines are replaced by fluorine, bromine or iodine. Methyl vinyl ketone (MVK) and MVK wherein some hydrogens are substituted with halogen e.g. 4,4-dichloro-3-butene-2-one, are also equivalent with respect to their reaction with thiocyanogen.

The treatment of polymers is ordinarily accomplished by immersing the polymer article (sheet or molved item) in a solution of the stable thiocyanogen reaction product for a period of time sufficient to wet the surface (usually 2-10 seconds in the case of polyurethanes). Other polymers may require longer periods of immersion. The wetted polymer article is then removed and allowed to dry at ambient temperature or placed in an oven to remove the solvent and/or to complete the reaction of the thiocyanogen product with the surface of the article. Temperatures of from ambient up to the boiling point of the solvent may be used in treating the article while those employed in evaporating the solvent from the surface may be from room temperature up to 150°C.

The stable reaction product produces thiocyanate moieties or functionality in the surface of the polymer article. Whether it decomposes or reacts by itself with the surface is not known, but it produces a more adherent surface than a thiocyanogen does.

Example 2

Use of the Stable Thiocyanogen-o-Chloranil Reaction Product in the Modification of Polyurethane

To a carbon tetrachloride solution of thiocyanogen prepared as in Example 1 was added 7.5 g (0.03 mole) of o-chloranil. This solution was then used to treat a specimen (1/2″ X 8″ X 1/8″ dimensions, or 12.7 mm x 203.2 mm x 3.2 mm) of reaction injection molded (RIM) polyurethane by immersing it in the solution for 5 seconds at room temperature. The polyurethane specimen was then placed in an oven heated to 130-140°C for twenty minutes. After cooling the specimen, the contact angle of 2-ethoxyethylacetate on the surface was measured. The average contact angle of 5 treated samples was 6°±1°, while for 5 untreated samples the contact angle was 26°±2°. The contact angle for treatment with freshly prepared thiocyanogen was 7°± 2°. The more wettable surfaces gave a smaller contact angle.

The procedure for measuring the contact angle was as follows:

Procedure for Measuring Contact Angle

1. Place polymer specimen to be evaluated in a Kayeness, Inc. Contact Angle Viewer, Model D-1060.
2. Place a drop of an appropriate liquid on the specimen from a height of one inch (25.4 mm).
3. Rotate the protractor so that the 90/90 vertical line is tangent to the arc at theleading edge of the drop. Read the contact angle at the arrow point.
4. Repeat 3 at the other edge of the drop.
5. Average the two readings.
6. Repeat steps 2, 3, 4, and 5, four times.
7. Average the four values to obtain the experimental contact angle.

Example 3

Isolation of the Stable Reaction Product of Thiocyanogen and o-Chloranil

Into a flask containing a magnetic stirring bar was placed 200 ml of a stabilized methylchloroform. To this was added 10.0 grams of potassium thiocyanate (KSCN) with stirring. Next, 200 milliliters of stabilized methylchloroform containing 1.4 g of chlorine, $Cl_2$, was added to the stirred mixture. Stirring was continued for one hour or until the odor of chlorine was not detected in the vapor space of the flask. Precipitated KCl solids were separated from the reaction mixture by filtration. Then, 9.9 grams of o-chloranil was added to the filtered thiocyanogen solution while agitating. The solution was allowed to stand for 48 hours.

The above prepared solution was poured into a large boiling flask and distilled under vacuum at 40°C to isolate the reaction product of thiocyanogen and o-chloranil. A portion (0.3046 g) of the solid dark red reaction product was dissolved in 25 ml of methanol. This solution was used to treat a piece of RIM polyurethane as described in Example 2. The contact angle of 2-methoxyethanol on the treated RIM was measured. A contact angle of 17° for the treated RIM was found as compared to a contact angle of 44° for untreated and a contact angle of 43° for RIM treated with methanol only.

Infrared spectroscopy of thiocyanogen solutions in $CCl_4$ normally shows a sharp characteristic peak at 2160 $cm^{-1}$ which on standing decreases rapidly over a period of 24 hours, presumably due to the formation of the inert thiocyanogen polymer.

A solution of thiocyanogen and o-chloranil in $CCl_4$ as prepared in Example 1 was examined by infrared spectroscopy. While the peak characteristic of thiocyanogen was apparent, another larger peak was observed at 2000 cm$^{-1}$. After 24 hours, reexamination of the CCl$_4$ solution of the product showed that the peak at 2000 cm$^{-1}$ had greatly increased, while that at 2160 cm$^{-1}$ had decreased. The absorbance at 2000 cm$^{-1}$ is characteristic of an organic isothiocyanate (R-N=C=S). Organic thiocyanates on the other hand (R-S-C≡N) exhibited weak, but sharp absorption at 2160 cm$^{-1}$.

In another experiment conducted in the manner of Example 1, o-chloranil and (SCN)$_2$ were reacted in CCl$_4$ solution at a 5:1 molar ratio, respectively. The solution was bottled, sealed and observed periodically. Only a very slight amount of precipitate was observed after 17 months of storage at ambient conditions.

## Example 4

Preparation of the Reaction Product of Thiocyanogen Chloride (SCNCl) and o-Chloranil

To 100 ml of CCl$_4$ containing 13 g of Pb(SCN)$_2$ was added 5.4 g of bromine with stirring, which was continued until the reaction mixture no longer showed the bromine color. The solution was filtered to remove PbBr2 and a clear yellow solution of (SCN)$_2$ was obtained. Then 100 ml of CCl$_4$ containing 2.4 g chlorine was added with stirring to the (SCN)$_2$ solution. The color of the solution changed to a deeper gold color which is typical of thiocyanogen chloride (SCNCl). A quantity (25 ml) of this solution was added to 75 ml of CCl$_4$ which contained 2.5 g o-chloranil. Another 25 ml of the solution of SCNCl was added to 75 ml of CCl$_4$ without o-chloranil. Both solutions were bottled and sealed and observed periodically. Thiocyanogen chloride is more stable than thiocyanogen, but after 64 days a precipitate had formed in the bottle without o-chloranil. After 76 days the bottle containing the reaction product of SCNCl and o-chloranil showed no precipitate and remained clear. The molar ratio of o-chloranil to SCNCl in the example was 1.2:1.

## Example 5

Preparation of the Reaction Product of Thiocyanogen with Methyl Vinyl Ketone

To a quantity of thiocyanogen (0.005 mole) solution in 100 ml stabilized methylchloroform was added 0.83 ml (0.01 mole) of methyl vinyl ketone. The solution at room temperature was stirred, bottled and sealed. The sample was observed periodically and there was no precipitate and sample was clear after 510 days (17 months) storage at ambient temperature. The molar ratio of methyl vinyl ketone to thiocyanogen was 2:1.

To show that the new composition reacts with organic compounds to form organic thiocyanates the following experiment was conducted.

## Example 6

Reaction with an Organic Cycloaliphatic Compound

To 15 ml of reagent grade cyclohexene in a clear glass container was added 25 ml of a solution of the reaction product of o-chloranil and thiocyanogen prepared as in Example 1.

The mixture was exposed to UV-light for 20 minutes while stirring. The reaction products as determined by gas chromatography/mass spectrometry were: dithiocyanato cyclohexanes, thiocyanato or isothiocyanato cyclohexenes, chlorothiocyanato cyclohexane, chlorocyclohexenes.

It should be noted that when halogen-containing α,β-unsaturated ketones are used that halothiocyanato products are also obtained. If one does not want a halogen-containing product, a non-halogen-containing α,β-unsaturated ketone should be used. Likewise, the use of a thiocyanogen halide will produce a halogen containing product.

## Claims

1. A composition comprising the reaction product of thiocyanogen or a thiocyanogen halide and an α,β-unsaturated ketone selected from 1,2-benzoquinone, methyl vinyl ketone or halo-derivatives of 1,2-benzoquinone or methyl vinyl ketone.

2. A composition as claimed in claim 1 wherein the α,β-unsaturated ketone is methyl vinyl ketone.

3. A composition as claimed in claim 1 wherein the haloderivative is 3,4,5,6-tetrachloro-1,2-benzoquinone.

4. A composition as claimed in any of the preceding claims wherein the reaction product is present in sol-

EP 0 239 790 B1

ution in an inert solvent.

5. A composition as claimed in claim 4 wherein the inert solvent is an aliphatic or aromatic hydrocarbon.

6. A method of stabilizing thiocyanogen or thiocyanogen chloride which comprises reacting thiocyanogen or thiocyanogen chloride with an $\alpha,\beta$-unsaturated ketone in an inert solvent wherein the $\alpha,\beta$-unsaturated ketone is selected from 1,2-benzoquinone, methyl vinyl ketone or halo-derivatives of 1,2-benzoquinone or methyl vinyl ketone.

7. The method of claim 6 wherein the halo-derivative is tetrachloro-1,2-benzoquinone.

8. A method of thiocyanating the surface of a polymer which contains unsaturation comprising contacting said surface with a solution of a stable reaction product of claim 1.

**Patentansprüche**

1. Zusammensetzung, die das Reaktionsprodukt von Thiocyanogen oder einem Thiocyanogenhalogenid und einem $\alpha,\beta$-ungesättigten Keton, ausgewählt aus 1,2-Benzochinon, Methylvinylketon oder Halogenderivaten von 1,2-Benzochinon oder Methylvinylketon, umfaßt.

2. Zusammensetzung nach Anspruch 1, worin das $\alpha,\beta$-ungesättigte Keton Methylvinylketon ist.

3. Zusammensetzung nach Anspruch 1, worin das Halogenderivat 3,4,5,6-Tetrachlor-1,2-benzochinon ist.

4. Zusammensetzung nach einem der vorhergehenden Ansprüche, worin das Reaktionsprodukt gelöst in einem inerten Lösungsmittel vorliegt.

5. Zusammensetzung nach Anspruch 4, worin das inerte Lösungsmittel ein aliphatischer oder aromatischer Kohlenwasserstoff ist.

6. Verfahren zum Stabilisieren von Thiocyanogen oder Thiocyanogenchlorid, umfassend das Umsetzen von Thiocyanogen oder Thiocyanogenchlorid mit einem $\alpha,\beta$-ungesättigten Keton in einem inerten Lösungsmittel, wobei das $\alpha,\beta$-ungesättigte Keton aus 1,2-Benzochinon, Methylvinylketon oder Halogenderivaten von 1,2-Benzochinon oder Methylvinylketon ausgewählt wird.

7. Verfahren nach Anspruch 6, worin das Halogenderivat Tetrachlor-1,2-benzochinon ist.

8. Verfahren zum Thiocyanatieren der Oberfläche eines Polymers, das Unsättigung enthält, umfassend das Kontaktieren der Oberfläche mit einer Lösung eines stabilen Reaktionsprodukts nach Anspruch 1.

**Revendications**

1. Composition comprenant le produit de réaction du thiocyanogène ou d'un halogénure de thiocyanogène et d'une cétone $\alpha,\beta$-insaturée choisie parmi la 1,2-benzoquinone, la méthylvinylcétone ou les dérivés halogénés de 1,2-benzoquinone ou de méthylvinylcétone.

2. Composition selon la revendication 1, dans laquelle la cétone $\alpha,\beta$-insaturée est la méthylvinylcétone.

3. Composition selon la revendication 1, dans laquelle le dérivé halogéné est la 3,4,5,6-tétrachloro-1,2-benzoquinone.

4. Composition selon l'une quelconque des revendications précédentes, dans laquelle le produit de réaction est présent en solution dans un solvant inerte.

5. Composition selon la revendication 4, dans laquelle le solvant inerte est un hydrocarbure aliphatique ou aromatique.

6. Procédé de stabilisation du thiocyanogène ou du chlorure de thiocyanogène, qui comprend la réaction du thiocyanogène ou du chlorure de thiocyanogène avec une cétone $\alpha,\beta$-insaturée dans un solvant inerte, dans lequel la cétone $\alpha,\beta$-insaturée est choisie parmi la 1,2-benzoquinone, la méthylvinylcétone ou les dérivés halogénés de 1,2-benzoquinone ou de méthylvinylcétone.

7. Procédé selon la revendication 6, dans lequel le dérivé halogéné est la tétrachloro-1,2-benzoquinone.

8. Procédé pour thiocyanater la surface d'un polymère qui contient une insaturation, comprenant la mise en contact de ladite surface avec une solution d'un produit de réaction stable selon la revendication 1.